# EUROPEAN PATENT APPLICATION

(11) **EP 1 170 018 A1**
(43) Date of publication of application: **09.01.2002**
(21) Application number: 00909640.5
(22) Date of filing: 15.03.2000
(51) Int. Cl.: A61K 39/44, A61K 47/48, A61P 35/00

(54) **LIGAND-BONDED COMPLEX**

(30) Priority: 17.03.1999 JP 7169099
(71) Applicant: MITSUBISHI CHEMICAL CORPORATION, Chiyoda-ku, Tokyo 100-0005 (JP)
(72) Inventor: TAGAWA, Toshiaki, Mitsubishi-tokyo Pharm., Inc., Yokohama-shi, Kanagawa 227-0502 (JP); SUZUKI, Tsutomu, Machida-shi, Tokyo 194-0042 (JP); YADA, Nobuhisa, Yokohama Research Center, Yokohama-shi, Kanagawa 227-0502 (JP); NAGAIKE, Kazuhiro, Mitsubishi Chemical Corp., Yokohama-shi, Kanagawa 227-8502 (JP); HIRAKAWA, Youko, Mitsubishi-Tokyo Pharm.,Inc., Yok ohama-shi, Kanagawa 227-0033 (JP); HOSOKAWA, Saiko, Mitsubishi-Tokyo Pharm.,Inc., Yokohama-shi, Kanagawa 227-0033 (JP)
(74) Representative: TER MEER STEINMEISTER & PARTNER GbR
(86) International application number: JP0001563
(87) International publication number: WO0054807

(57) **Abstract**

A ligand-bonded complex which does not react with a free target such as a soluble tumor antigen, but enables substantially specific reaction with a non-free target such as a tumor cell and a tumor antigen existing in the cell.

## Description

### Technical Field

The present invention relates to a ligand-bonded complex. More specifically, the present invention relates to a ligand-bonded complex that does not substantially bind to a free target substance such as a free antigen, but can specifically react with a target such as a cancer cell.

### Background Art

Attempts have widely been made to cell-specifically or site-specifically direct a drug by utilizing a ligand having specific reactivity such as an antibody. Targeting therapies against cancer cells are examples of such attempts. Drugs have already been developed such as a complex of an antibody and a toxin, a complex of an antibody and a radioactive compound, and an immuno-liposomes and so forth. Diagnoses of cancers and the like in vivo have also been performed by using a complex of an antibody and a radioactive compound. Targeting therapies using such antibody-bonded complexes are based on high specificity of the ligand (antibody and the like) to a target, and therefore, superior therapeutic effect and reduced side effect can be expected.

In the targeting therapy, however, a problem has been pointed out in that an antibody-bonded complex reacts with a free target such as a free antigen existing in blood or the like, and thus a sufficient amount of the drug cannot react with a solid tumor tissue, including a primary lesion and a metastatic foci, having non-free antigens and the like. In other words, when a part of antigens are secreted into blood or antigens are released from cancer cells and free antigens (soluble antigens) appear in blood, as observed in certain types of cancers, antibody-bonded complexes will react with the free antigens to form immuno-complexes, thereby the reaction with target cells will be inhibited. Accordingly, in order to design an antibody-bonded complex, it is generally required to chose an antibody whose antigen is absent or extremely low level in blood. Furthermore, an antibody against an antigen, whose significance in serum diagnosis has been established clinically, is impossible to use in the manufacture of an antibody-bonded complex,.

### Disclosure of the Invention

In order to solve the aforementioned problem, the inventors of the present invention conducted researches on the relationship between soluble target substances such as a free antigen and ligands such as an antibody. Surprisingly, it was found that a ligand-bonded complex, to which plural numbers of a ligand having a low affinity to a target substance were bonded, had a high reactivity to a non-free target such as a cancer cell even in the presence of a free target substance. The present invention was achieved on the basis of these findings.

The present invention thus provides a ligand-bonded complex which does not substantially bind to a free target substance such as a free antigen, but can specifically react with a non-free target such as a cancer cell. More specifically, the present invention provides a ligand-bonded complex in which a ligand having affinity for a target is bonded directly or indirectly to a microparticle, wherein the affinity of the ligand is sufficient to allow substantially specific binding of the ligand-bonded complex to a non-free target even in the presence of a free target; the aforementioned complex wherein two or more of a single kind of a ligand having substantially the same affinity are bonded to one microparticle; and the aforementioned complex wherein an amount of the ligand is sufficient for reaction of the ligand with the non-free target.

There are also provided the aforementioned complex wherein the ligand is directly bonded to the microparticle; the aforementioned complex wherein a water-soluble macromolecule is bonded to the microparticle; and the aforementioned complex wherein a part of or all of the molecules of the ligand are indirectly bonded to the microparticle, preferably the aforementioned complex wherein a part of or all of the molecules of the ligand molecules are indirectly bonded to the microparticle by means of a water-soluble macromolecule.

There are further provided the aforementioned complex wherein the water-soluble macromolecule is a polyalkylene glycol, preferably polyethylene glycol; the aforementioned complex wherein the microparticle is selected from the group consisting of a low molecular drug, a marker molecule, a protein, a micelle, and a liposome; the aforementioned complex wherein the microparticle is a liposome; the aforementioned complex wherein the liposome encapsulates an active principle of a medicament; the aforementioned complex wherein the medicament is an anti-tumor agent; the aforementioned complex wherein the liposome encapsulates an anti-tumor agent, preferably adriamycin or the like; and the aforementioned complex wherein the ligand is an antibody, preferably an anti-tumor antibody, more preferably a human cancer cell-reactive human monoclonal antibody.

There are also provided the aforementioned complex wherein dissociation constant between the target substance and one ligand is E-8 M or more, preferably E-7 M or more; and a pharmaceutical composition containing the aforementioned complex.

In the ligand-bonded complex according to a more preferred embodiment of the present invention, the microparticle is a liposome encapsulating adriamycin, polyethylene glycol is bonded to a surface of the liposome, and an anti-tumor antibody, preferably a human cancer cell-reactive human monoclonal antibody, having the aforementioned affinity is bonded to a part of the polyethylene glycol molecules. Preferably, two or more of the anti-tumor antibodies (plural antibodies), sufficient amount for reaction with a non-free target, are bonded per one liposome, and the antibodies are bonded to end portions of the polyethylene glycol molecules.

### Brief Explanation of the Drawings

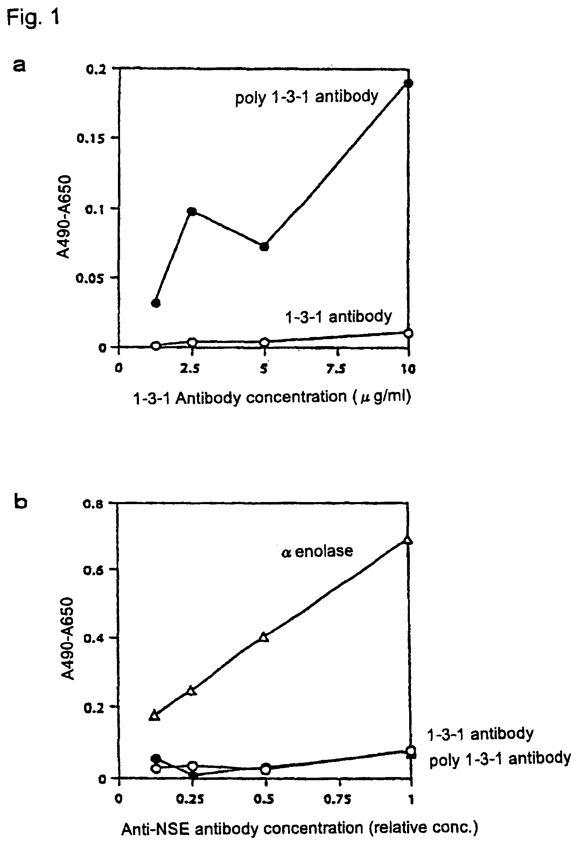

Fig. 1 shows results of enzyme immunoassay utilizing 1-3-1 antibodies and poly 1-3-1 antibodies. In the figure, the horizontal axis indicates antibody concentration, and the longitudinal axis indicates absorbance obtained by using OPD as a substrate. Fig. 1-a shows the results obtained when antigens were immobilized, and Fig. 1-b shows the results obtained when antibodies were immobilized.

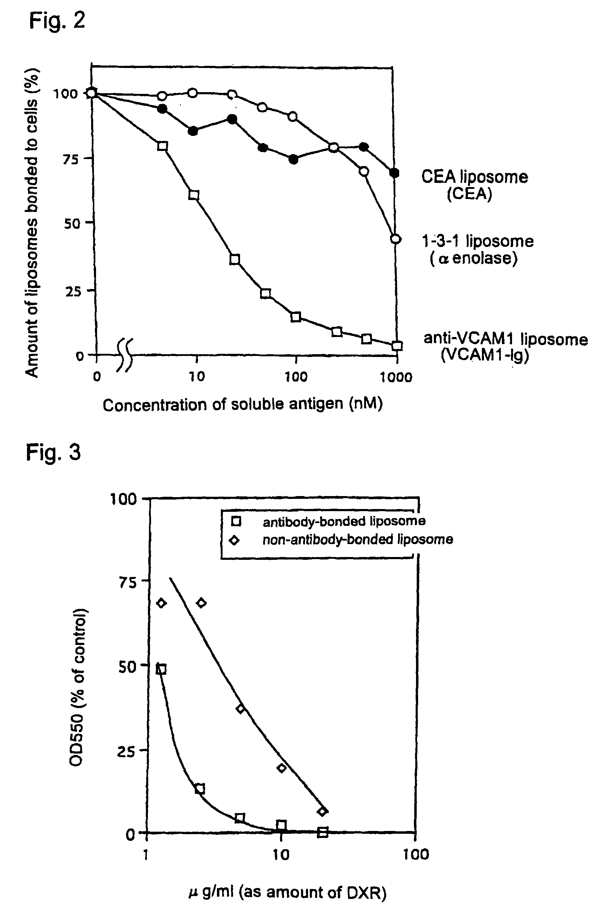

Fig. 2 shows results obtained from reactions of various kinds of ligand-bonded liposomes encapsulating fluorescent dyes with target cells in the presence of free antigens. In the figure, the horizontal axis indicates concentration of soluble antigen (free antigen) in a reaction mixture, and the longitudinal axis indicates amount of liposomes bonded to the cells. The amount is indicated as a relative amount based on the amount of liposomes bonded to the cells in the absence of soluble antigens, which is taken as 100.

Fig. 3 shows in vitro anti-tumor effect of 1-3-1 antibody-bonded immuno-liposomes encapsulating adriamycin (DXR) on the human colon cancer cell strain DLD-1. In the figure, the horizontal axis indicates amount of liposomes as amount of DXR, and the longitudinal axis indicates ratio of cell number at each liposome concentration relative to the cell number obtained when liposomes were not added, which is taken as 100.

### Best Mode for Carrying out the Invention

Type of the ligand used for the present invention is not particularly limited so long as it has appropriate affinity for a target substance. For example, proteins such as transferrin, CEA, EGF and AFP; peptides such as insulin; antibodies such as monoclonal antibodies; antigens such as tumor antigens; hormones; transmitters; saccharides such as Lewis X and gangliosides; and low molecular compounds such as folic acid and derivatives thereof may be used. The ligands exemplified above may be used as a whole, or a fragment thereof obtained by an enzymatic treatment or the like may be used. Further, artificially synthesized peptides and peptide derivatives may also be used. When antibodies are used for therapeutic application, mouse-human chimeric antibodies, humanized antibodies, human antibodies and so forth are preferred. As the ligand, antibodies are preferred and anti-tumor antibodies are more preferred. Particularly preferred are human cancer cell-reactive human monoclonal antibodies, and further preferred is human cancer cell-reactive human monoclonal antibody 1-3-1 (see, Japanese Patent Unexamined Publication (Kokai) No. 5-304987).

The term "target substance" used in this specification means a substance to which a ligand can specifically bind. Its type is not particularly limited, and any low molecular substance or macromolecular substance may be used. As the target substance, examples include, for example, antigens, antibodies, receptors, growth factors and so forth. While the ligand and the target substance usually mean different molecules, a macromolecular substance having a property of binding to each other among the same molecules, for example, CEA as a fetal cancer antigen (it is considered that weak interaction of CEAs among themselves contributes to the cell adhesion) may be used as the ligand and the target substance. As the target, tumor antigens are preferred, and particularly preferred are tumor antigens of colon cancer, stomach cancer and so forth that can be recognized by the human cancer cell-reactive monoclonal antibody 1-3-1.

The term "non-free target" herein referred to is used to encompass cells, tissues and the like having a target substance to which the ligand binds.

The term "free target" herein used generally means a substance such as low molecular compounds, polypeptides and proteins which is released from a non-free target, existing in a state of a solid such as tumor cells and tumor tissues, into blood, lymph or the like, and can generally be recognized by the ligand at a substantially equivalent level as the non-free target. Typical free targets are soluble antigens released from tumor cells into blood. The free target may be a substance identical to a cell surface antigen existing in the non-free target, or an analogous molecular species having the same epitope. According to the present invention, plural numbers of the ligand are bonded to the surface of the microparticle, thereby its apparent affinity is increased. Accordingly, they specifically binds to the "non-free target", but substantially does not specifically binds to the "free target".

The ligand used for the ligand-bonded complex of the present invention must have an affinity to the target substance such that the ligand-bonded complex can substantially specifically bind to a non-free target in the presence of a free target. Whether or not a ligand has such affinity as described above can be easily determined by preparing the ligand-bonded complex and then investigating its binding ability to a non-free target in the presence of a free target according to the method specifically explained in the examples mentioned in the present specification.

In order to control degree of affinity of the ligand, the ligand may be chemically modified to alter a part of its structure, or when the ligand is an antibody, protein, peptide or the like, an amino acid mutation may be introduced by a genetic engineering technique. A modified substance such as a single chain antibody can also be used. For example, those showing a dissociation constant between the ligand and the target substance of about E-8 (M) or more, preferably about E-7 (M) or more, are preferred.

As the method of binding plural molecules of the ligand to the microparticle, a method of crosslinking the ligand molecules may be used. Such crosslinking can be attained by an ordinary method utilizing a crosslinking agent. For example, applicable crosslinking methods include the glutaraldehyde method, the periodic acid method, the maleimide method and the pyridyldisulfide method (Enzyme Immunoassay, Eiji Ishikawa et al., Igaku-Shoin). It is also possible to bind a toxin, a protein, a drug, a radioactive element or the like to the crosslinked ligand such as antibodies. For example, radioactive iodine can be introduced into an antibody using the chloramine-T method. It is also possible to introduce ¹¹¹In or the like by using a divalent chelating reagent such as aminobenzyl-EDTA and isothiocyanobenzyl-EDTA (Nuclear Medicine, 31, 473 (1994)).

It is known that properties of a liposome can be changed by binding a water-soluble macromolecule to the surface of the liposome. Such a water-soluble macromolecule can be bound to the surface of the microparticle of the ligand-bonded complex of the present invention. A suitable water-soluble macromolecule can be chosen depending on desired properties. For example, synthetic macromolecules such as polyalkylene glycols, polyacrylamides, polyvinylpyrrolidones, polyglycerols, polylactic acids, polyglycolic acid, polyamino acids and the like may be used. Further, biodegradable polymers such as polyamino acids and polyoxy acids are also suitably used. The water-soluble macromolecule preferably has a molecular weight of about 500-20,000, more preferably 1,500-10,000, further preferably 2,000-6,000. As the water-soluble macromolecule, a polyalkylene glycol, more preferably polyethylene glycol can be used.

When the water-soluble macromolecule is bonded to the surface of the microparticle, a part of or all of the ligand molecules can be bonded to a part of or all of the water-soluble macromolecules. For example, an embodiment in which all of the ligand molecules are bonded to a part of polyalkylene glycol molecules that are bound to the surface of the microparticle is preferred according to the present invention. In this embodiment, it is more preferred to bond the ligand molecules at the ends of the polyalkylene glycol molecules. Further, the ligand can be introduced into a polyamino acid such as polylysine and polyaspartic acid by means of an amide bond. Radioisotopes, toxic proteins, drugs and the like may further be introduced into the aforementioned ligand.

As the microparticle that constitutes the ligand-bonded complex of the present invention, there can be used, for example, micelles obtained by aggregation of amphiphilic molecules containing a hydrophilic moiety and a hydrophobic moiety in the molecule, macromolecular micelles, microspheres, emulsions, liposomes, polymer vesicles obtained by polymerization of bilayer vesicles such as liposomes, cationic liposomes, gene complexes and so forth. The microparticle may be in a shape of a small sphere, an ellipse, or a long cylinder constituted by an amphiphilic substance. For example, the microparticle may be a natural vesicle such as cells and viruses, modified natural vesicle such as those obtained by radiation irradiation or modification with a macromolecule of natural vesicles, liposomes, novasomes, non-surfactant vesicles and the like. The diameter of the microparticle may be, for example, about 20-500 nm.

As the microparticle, a liposome can be suitably used. A type of the liposome is not particularly limited, and any of multilamellar liposome (MLV), small unilamellar liposome (SUV) and large unilamellar liposome (LUV) may be used. LUV can be preferably used.

As for the amphiphilic molecule constituting the microparticle, its type is not limited so long as it contains a hydrophilic moiety and a hydrophobic moiety and can form the microparticle. An example of preferred amphiphilic substance includes lipids. Examples of the lipids include phospholipids, sphingoglycolipids, glycoglycerolipids and the like, for example, natural phosphatidylcholines such as egg yolk phosphatidylcholine (EYPC); phosphatidylcholines (PC) such as dipalmitoylphosphatidylcholine (DPPC), dimyristoylphosphatidylcholine (DMPC), distearoylphosphatidylcholine (DSPC) and dioleoylphosphatidylcholine (DOPC); natural phosphatidylethanolamines such as egg yolk phosphatidylethanolamine (EYPC); phosphatidylethanolamine (PE) such as dipalmitoylphosphatidylethanolamine (DPPE), dioleoylphosphatidylethanolamine (DOPE) and dimyristoylphosphatidylethanolamine (DMPE); phosphatidylglycerols (PG) such as dipalmitoylphosphatidylglycerol (DPPG); phosphatidylserine (PS); phosphatidylinositol (PI); phosphatidic acid (PA) such as dipalmitoylphosphatidic acid. These lipids may be used alone or in combination of two or more kinds, or they may be used in combination with a non-polar substance such as cholesterols. Furthermore, a charged substance including stearylamine, dicetylphosphate and cholesterol derivatives such as DC-Chol (3 β -[N-(N',N'-dimethylaminoethyl)carbamoyl]-cholesterol), phospholipid derivatives having a maleimide group (Japanese Patent Unexamined Publication (Kokai) No. 6-157559), phospholipid derivatives having a maleimide group at the end of PEG (Japanese Patent Unexamined Publication (Kokai) No. 6-220070) and the like may be incorporated. The microparticle may be incorporated with a part or whole of a virus such as, for example, a liposome fused with Sendai virus known as a fusiogenic liposome.

Methods for producing the microparticle such as micelles or liposomes are not particularly limited and any method available to those skilled in the art can be employed. For example, usable methods include a method of producing MLV by adding an aqueous solution to a thin lipid membrane attached to a glass wall and subjecting the system to mechanical shaking; a method of producing SUV by the sonication method, the ethanol injection method, or the French press method; a method of producing LUV by the detergent removing method, the reversed phase evaporation method (Liposome, Sunamoto et al., Nankodo, 1998), the extrusion method in which MLV is extruded through a membrane having a uniform pore size under positive pressure or the like (Liposome Technology, vol.1, 2nd edition).

Type of the drug encapsulated in the microparticle is not particularly limited, and an active principle of any drug for therapeutic and prophylactic treatments and diagnosis can be encapsulated in the microparticle. Examples include anti-tumor agents such as adriamycin (doxorubicin, DXR), daunomycin, vinblastine, cisplatin, mitomycin, bleomycin and 5-FU (5-fluorouracil); adrenalin blockers such as timolol; antihypertensive agents such as clonidine; antiemetic agents such as procainamide; antimalarial agents such as chloroquinine; antibiotics such as amphotericin; toxic proteins such as ricin A chain and diphtheria toxin as well as genes encoding the proteins; antisense genes such as k-ras, genes encoding TNF, P53 and the like as well as complexes of these genes and polycations such as polylysine; radioisotopes of iodine, rhenium, indium, technetium, yttrium and so forth; MRI contrast agents such as gadolinium; X-ray imaging contrast agents such as iodine compounds; ultrasonic imaging contrast agents such as CO₂; fluorescent substances such as europium and carboxyfluorescein; luminescent substances such as N-methylacridium derivatives; enzymes such as horseradish peroxidase and alkaline phosphatase and so forth.

Methods for introducing these drugs into the microparticle are not particularly limited, and any method available to those skilled in the art can be employed. For example, when a liposome is used as the microparticle, an aqueous solution of an active principle can be added during the formation of the liposome so as to be encapsulated in the liposome. Further, after the formulation of the liposome, a method can be used in which a concentration gradient such as a pH gradient is formed between inside and outside of the vesicle and this potential is used as a driving force to take up an ionizable drug into the liposome (Cancer Res., 49, 5922 (1989); BBA, 455, 269 (1976)).

Means for bonding the ligand to the microparticle are not particularly limited, and the bonding may be formed by any means including covalent bond, ionic bond and the like. For example, when a liposome is used as the microparticle, a reactive group that can react with the ligand such as maleimide group and carboxyl group can be introduced into the liposome, and the ligand can be bonded to the liposome after the formation of the liposome (Japanese Patent Unexamined Publication (Kokai) Nos. 6-157559 and 6-220070; Advanced Drug Delivery Reviews, 24, 235 (1997)). More specifically, a liposome having a maleimide group moiety can be produced by forming a liposome using a lipid having a maleimide group moiety such as maleimidocaproyldipalmitylphosphatidylethanolamine (Japanese Patent Unexamined Publication (kokai) No. 4-346918) and maleimidophenylbutyroylphosphatidyl-ethanolamine together with phosphatidylcholine and cholesterol according to a known method (Liposome, Chapter 2, edited by Nojima et al., Nankodo (1988)).

Further, a hydrophobic compound such as lipids can be introduced into the ligand beforehand, and the ligand can be introduced into a liposome by the surfactant removing method at the time of formation of the liposome (BBA, 1070, 246 (1991)). The ligand may be directly bonded to the microparticle, or the ligand may be indirectly bonded to the microparticle by means of a water-soluble macromolecule as a spacer as described above. As the water-soluble macromolecule that can be used as a spacer, for example, the water-soluble macromolecule derivatives disclosed in Japanese Patent Application No. 10-263262 can be used. After the ligand is bonded to the microparticle, it is also possible to further modify the surface of the microparticle with a water-soluble macromolecule as required.

The ligand-bonded complex of the present invention can be used for therapeutic or prophylactic treatments or diagnosis of target diseases depending on the type of a drug encapsulated in the microparticle. A route of administration and an administration dose can be suitably chosen according to the type of an encapsulated drug, properties of a microparticle and a purpose of administration. It is generally desirable that the complex is used via an administration route such as intravascular administration, intravesical administration, intraperitoneal administration, local administration or the like.

According to a particularly preferred embodiment of the ligand-bonded complex of the present invention, a liposome encapsulating adriamycin is used as the microparticle, and polyethylene glycol molecules are bonded to the surface of the liposome. Further, anti-tumor antibodies are bonded to end portions of some of the polyethylene glycol molecules, so that plural antibodies per liposome, i.e., antibodies in an amount sufficient for reaction with a non-free target, are bonded. The antibodies have such affinity that the ligand-bonded complex does not substantially react with a free tumor antigen existing in blood, but specifically reacts with a non-free target having a tumor antigen (tumor cell or tumor tissue).

The ligand-bonded complex according to the aforementioned preferred embodiment is useful for targeting therapy of tumors, and it can be used by intravascular administration, intravesical administration, intraperitoneal administration, or local administration. A dose may be 10 mg/kg or less, preferably 5 mg/kg or less, more preferably 1 mg/kg or less as an amount of adriamycin. Applicable tumors are not particularly limited. The complex may preferably be applied to types of tumors whose tumor antigens are available, for example, those among solid tumors including stomach cancer, colon cancer, esophagus cancer, oral cavity cancer, liver cancer, kidney cancer, breast cancer, ovary cancer, uterus cancer, prostate cancer, lung cancer, brain tumor and so forth,. The method of producing a monoclonal antibody that specifically recognizes a tumor antigen is well known to those skilled in the art, and a monoclonal antibody that has the affinity defined in the present specification can be suitably selected.

### Examples

The present invention will be more specifically explained with reference to the following examples. However, the scope of the present invention is not limited by these examples.

### Example 1

### (1) Dissociation constant of 1-3-1 antibody

Antibody-bonded liposomes were produced by using an F(ab')² fragment (molecular weight: 100 kDa) of human cancer cell-reactive human monoclonal antibody 1-3-1 (IgG).

This antibody is reactive to human enolase (α and γ) and human stomach cancer cell MKN45. The antibody was labeled with FITC, and its dissociation constant to MKN45 immobilized with paraformaldehyde was measured by using a flow cytometer (FAC Svantage, Becton Dickinson), which was found to be 1E-7 (M). The dissociation constant was obtained by kinetic analysis of bonding reaction and dissociation reaction during the reaction as follows.

### Bonding reaction:

MKN45 cells immobilized with paraformaldehyde were suspended in 500 µL of 1% BSA solution. FITC-labeled antibodies were accurately quantified so that a final concentration of the antibodies was 1-10 µg/mL, and placed in a tube. After the temperature of the mixture was adjusted to 25°C, the antibodies and the cells were mixed immediately by using Vortex provided on a sample port, and simultaneously the cells were flown to start the measurement. Since the flow cytometer can detect only the fluorescence bonded to cells, it enables measurement of only a degree of shift caused by the antibodies bonded to cells even in the presence of free antibodies. After the measurement was started, the degree of shift caused by binding of the antibodies was measured at intervals of 5 seconds to 10 seconds and converted into a value of fluorescence intensity based on a calibration curve obtained by using fluorescent latex beads having a known fluorescence intensity (Ortho Diagnostic Systems). The value of the fluorescence was further converted into the amount of antibodies by dividing the value with the number of the fluorescent molecules introduced into one antibody molecule. The amount of antibodies "Ft" bonded to the cells at a measurement time "t" (an amount of antigen/antibody complexes) was measured under the condition of an initial concentration "C" of each antibody.

Reaction rate constant of the binding of the antibody to the antigen, kass, was obtained as follows. The binding reaction rate is proportional to the concentrations of the antigen and the antibody, and expressed as kass*C*(Fmax - Ft) by using the binding reaction rate constant kass. Similarly, the dissociation reaction rate is expressed as kdiss*Ft by using the dissociation reaction rate constant, kdiss. If an initial antibody concentration providing excessive antibodies with reference to the antigen/antibody complexes is applied, a rate equation: dFt/dt = kass*C*(Fmax - Ft)-kdiss*Ft can be obtained. This equation can be further transformed into dFt/dt = kass*C*Fmax - (kass*C + kdiss). After regression of a curve plotted Ft versus time (t) for each antibody concentration, when the F value for each time is plotted versus the derivative dF/dt, the plot is obtained as a primary function and its slope is represented as - (kass*C + kdiss). Thus, the slope for each concentration C was calculated. Since the relationship between C and - (kass*C + kdiss) is also represented as a primary function, the function was plotted and kass was calculated from the slope of the line.

### Dissociation reaction:

The cells were reacted with the fluorescence-labeled antibodies at each concentration, and then washed by centrifugation to remove free antibodies. The cells in the form of pellet obtained by centrifugation was added with 500 µL of 1% BSA solution, which was warmed to 25°C beforehand, and then the amount of fluorescence bonded to the cells was immediately measured by using a flow cytometer. The amount of the bonded antibodies Ft was measured at each time point for each antibody concentration as described above, and the dissociation reaction rate constant kdiss was calculated as follows. Since the antibody concentration C is 0, the aforementioned equation is represented as dFt/dt = -kdiss*Ft. By obtaining a definite integral from time t1 to time tn when solving the above differential equation, a relationship ln(Ft1/Ftn) = kdiss*(tn - t1) can be obtained. Therefore, by plotting tn-t1 and ln(Ft1/Ftn), kdiss was obtained from the slope of the plot. Based on the above results, the dissociation constant Kd was calculated according to an equation Kd = kdiss/kass.

### (2) Preparation of 1-3-1 antibody polymer and its reactivity to non-free antigen and free antigen

To the aforementioned F(ab')₂ antibodies dissolved in 50 mM phosphate buffer, 1 mM EDTA, pH 7.0 (1.3 mg/ml, 1.5 ml), 18 µL of S-acetylthioglycolic acid N-hydroxysuccinimide ester (Sigma, also abbreviated as "SATA" hereinafter, 5 mg/ml) dissolved in dehydrated methanol was added and allowed to react at 25°C for 1 hour. After the buffer was exchanged with 50 mM phosphate buffer, 1 mM EDTA, pH 7.0 by using a PD-10 column (Pharmacia), 0.5 M hydroxylamine solution (0.5 M hydroxylamine, 0.5 M HEPES, 25 mM EDTA, pH 7.0) was added to the reaction mixture in an amount of 1/9 volume of the antibody solution. After deacetylation by a reaction at 25°C for 10 minutes, desalting was performed and the buffer was exchanged by using a PD-10 column equilibrated with 0.1 M phosphate buffer, 1 mM EDTA, pH 6.0 to obtain antibodies introduced with thiol groups.

To the aforementioned F(ab')₂ antibodies dissolved in 50 mM phosphate buffer, 1 mM EDTA, pH 7.0 (1.3 mg/ml, 1.5 ml), 18 µL of N-(ε-maleimidocaproyloxy)-succinimide (Dojin Kagaku, 5 mg/ml) dissolved in dehydrated methanol was added and reacted at 25°C for 1 hour. The buffer was exchanged with 50 mM phosphate buffer, 1 mM EDTA, pH 7.0 by using a PD-10 column to obtain 1-3-1 antibodies introduced with maleimide groups. Equal amount of the aforementioned antibodies introduced with thiol group and the antibodies introduced with maleimide groups were mixed and reacted at 25°C for 2 hours to obtain 1-3-1 antibody polymer (poly 1-3-1). The reactivity of this antibody was verified by the following enzyme immunoassay (EIA).

### Reactivity to non-free antigen:

α-Enolase was dissolved in 50 mM carbonate buffer (pH 9.6) at a concentration of 50 µg/mL, added to a 96-well plate and immobilized on the plate through a reaction at 37 °C for 2 hours. After the plate was blocked with 0.5% gelatin, the 1-3-1 antibody and the poly 1-3-1 antibody were added to the plate at various dilution rates. After a reaction at 37°C for 1 hour, the plate was washed with PBST (PBS containing 0.05% Tween 20), and then anti-human Igs-HRP (immunized animal was goat, Capel) was added as a secondary antibody to each well and the mixture was reacted at 37°C for 1 hour. After the plate was washed with PBST, antibodies reacted with the α-enolase immobilized on the plate were detected by using OPD as a substrate. The 1-3-1 antibody and the poly 1-3-1 antibody gave no difference in the reactivity to the secondary antibody. As a result, high reactivity was achieved by the poly 1-3-1 antibody as shown in Fig. 1-a.

### Reactivity to free antigen:

The poly 1-3-1 antibody and the 1-3-1 antibody were dissolved in 50 mM carbonate buffer (pH 9.6) at various concentrations, added to a 96-well plate and immobilized on the plate through a reaction at 37 °C for 2 hours. After the plate was blocked with 0.5% gelatin, 20 µg/mL of α -enolase was added. After the plate was washed, anti-enolase antibody (anti-NSE antibody, Biomeda, rabbit polyclonal antibody) were used as a secondary antibody, anti-rabbit IgG-HRP antibody was further used as a third antibody, and color development reaction was performed by using OPD in the same manner. As a result, reaction to the enolase added as a solution was scarcely observed in EIA using both of the immobilized antibodies (Fig. 1-b).

These results demonstrate that the poly 1-3-1 antibodies had much higher reactivity to the immobilized α-enolase compared with the 1-3-1 antibody, and both of the 1-3-1 antibody and the poly 1-3-1 antibody had substantially no reactivity to the free antigen. Therefore, it is suggested that, when a ligand-bonded complex is produced, the reactivity to an immobilized antigen can be enhanced and the reactivity to a free antigen can be reduced by binding plural antibody molecules, having such affinity as that of the 1-3-1 antibody, as ligands to a microparticle. It was verified that the α-enolase was detectable by the combination of 215M antibody and anti-rabbit IgG-HRP antibody by directly immobilizing the enolase on the plate as described above (Fig. 1-b).

The α-enolase used for the aforementioned test was purified from a culture supernatant of the human stomach cancer cell strain MKN45. The MKN45 cells were cultured without addition of serum, and 140 mL of the culture supernatant was concentrated by ultrafiltration (PM-10, Amicon), substituted with 0.1 M acetate buffer (pH 5.0), loaded on a cation exchange chromatography column Mono-S (Pharmacia), and eluted with a linear gradient of 0 M to 0.5 M NaCl concentration in the same acetate buffer. Fractions with peaks indicating reactivity to the 1-3-1 antibody were collected, concentrated, and then loaded on a YMC-PAC C4-AP column. The fractions were developed with a linear gradient of from water (containing 0.1% TFA) to acetonitrile (containing 0.08% TFA), and fractions of the main peak reactive to the 1-3-1 antibody were obtained. The peak was detected as a single band in SDS-PAGE. Peptide mapping and sequence analysis revealed that the peak was of α-enolase.

### Example 2: Production of 1-3-1 antibody-bonded liposome and binding test to cancer cell

### <Introduction of thiol group into antibody>

To the aforementioned F(ab')₂ antibodies dissolved in 50 mM phosphate buffer, 1 mM EDTA, pH 7.0 (1.4 mg/ml), 6.4 times in mole of SATA dissolved in dehydrated methanol was added and allowed to react at 25°C for 1 hour. The reaction mixture was adjusted to pH 4 with addition of 0.1 M acetic acid, and then loaded on a SP-Sepharose column (Pharmacia) equilibrated with 0.1 M acetate buffer, pH 4. After washing with the same buffer, the adsorbed antibodies were eluted with 50 mM phosphate buffer, 1 mM EDTA, pH 7.5. After the buffer was exchanged with 50 mM phosphate buffer, 1 mM EDTA, pH 7.0 by using Centricon 30 (Millipore), 0.5 M hydroxylamine solution (0.5 M hydroxylamine, 0.5 M HEPES, 25 mM EDTA, pH 7.0) was added to the reaction mixture in an amount of 1/9 volume of the antibody solution. After deacetylation through a reaction at 25°C for 10 minutes, desalting was performed and the buffer was exchanged by using an NAP-10 column (Pharmacia) equilibrated with 0.1 M phosphate buffer, 1 mM EDTA, pH 6.0 to obtain antibodies introduced with thiol groups. The introduced thiol groups were quantified by using 4,4'-dithiopyridine (Sigma) (Lecture of Biochemical Experiments, New Series", vol. 5, p.109, Ed. by Chemical Society of Japan). When calculation was performed by assuming that the molecular extinction coefficient of produced 4-pyridone was 22,500 and absorption at 280 nm of 1% solution of the antibodies was 14, the number of introduced thiol groups per F(ab')₂ molecule was 1.4.

### <Preparation of liposome>

To 400 mg of lipids consisting of uniformly mixed DPPC, cholesterol and ε-maleimidecaproyldipalmitoylphosphatidylethanolamine (MC-DPPE) (18:10:0.5 in molar ratio), 4 mL of 10 mM aqueous solution of carboxyfluorescein (CF) was added and mixed at 60°C by using a vortex mixer for hydration. Then, freeze and thawing of the mixture was repeated three times to produce multilamellar liposomes encapsulating CF. The liposomes were sized by successive extrusion from extruders provided with 0.1 µm and 0.2 µm Nucleopore membranes, respectively, to obtain CF-encapsulating liposomes.

### <Binding of antibody to liposome>

The aforementioned liposomes were diluted with 0.1 M phosphate buffer, 1 mM EDTA, pH 6.0 to a lipid concentration of 43 mg/mL. This liposome solution was added with the aforementioned thiolated antibodies in an amount of 8% by weight relative to the weight of lipids, and allowed to react at 25°C for 1 hour and further react at 10°C overnight. This reactant was purified by a Sepharose CL6B column equilibrated with physiological saline to obtain target 1-3-1 antibody-bonded immunoliposomes. Lipid concentration of the obtained immunoliposomes was measured by using Phospholipid C Test Wako (Wako Pure Chemical Industries). As for the amount of the antibody, the antibodies were solubilized with SDS and then the amount was quantified by using a BCA Kit (Pierce). As a result, the amount of bonded antibodies was 5% of the weight of the lipids.

### <Binding test to cancer cell>

Cells of the human stomach cancer cell strain MKN45 were hypodermically transplanted into a nude mouse, and a tumor tissue was removed when it became sufficiently large. The tumor tissue was chopped and filtered through a mesh to collect cancer cells, and the cells were fixed with paraformaldehyde. The 1-3-1 antibody-bonded liposomes were mixed with human neuron-specific enolase (γ-enolase, Advanced Immuno Chemical) at various concentrations in human blood serum (concentration of lipids of immunoliposomes: 100 µg/mL), and preincubated at 37°C for 30 minutes. The aforementioned cancer cells (E⁶ cells) made into a pellet was added with the above solution, suspended in the solution, and allowed to react at 37°C for 1 hour. After the cancer cells were washed with PBS containing 1% BSA, fluorescence of the immunoliposomes bonded to the cells was determined by using a flow cytometer. As a result, as shown in Fig. 2, almost no decrease in the reactivity to the cells was observed even when the concentration of the free antigen (soluble antigen) was increased.

### Comparative Example 1: Preparation of VCAM-1 antibody-bonded liposome and binding experiment to cancer cell

CF-including anti-VCAM-1 immunoliposomes were produced in the same manner as in Example 2 except that commercially available anti-human VCAM-1 mouse monoclonal antibodies BBIG-V1 (IgG, R & D Systems) were used. The dissociation constant of the antibody was E-9 M, and the amount of bonded antibodies was 1% by weight of the lipids. The influence of free antigen on the reaction of the immunoliposomes was investigated in the same manner as in Example 2, except that the following CHO cells introduced with human VCAM-1 was used as target cells of the CF-including anti-VCAM-1 immunoliposomes and human VCAM-Ig was used as the free antigen. As a result, as shown in Fig. 2, the reactivity of the immunoliposomes rapidly decreased in a free antigen concentration-dependent manner. The target cells and the free antigen used in this comparative example were prepared as follows.

### <Target cell>

Human VCAM-1 cDNA (R & D System) was subcloned into an expression vector pME18s, and co-transfected into CHO together with a resistance marker pSV2neo by using lipofectin to clone a G418 resistant strain as the target cell. The expression on of VCAM-1 on the cytoplasmic membrane of CHO was verified by flow cytometry.

### <Free antigen>

A fragment of human VCAM-1 cDNA (R & D System) for the extracellular region (1-698th amino acids) and a fragment for CH2 to CH3 regions of human IgG were ligated by PCR to obtain cDNA (VCAM-1 Ig). This cDNA was introduced into CHO by the aforementioned method to construct a VCAM-1 Ig secreting CHO cell, and secretion type ICAM-1 separated and purified from the cell culture solution was used as the free antigen.

### Example 3: Preparation of CEA-bonded liposome and binding experiment to cancer cell

CEA functions as a marker and also acts as an adhesion factor, and weak binding interaction among CEAs themselves is known. Influence of the free antigen on CEA-bonded liposomes was investigated. CF-including liposomes bonded with CEA were produced in the same manner as in Example 2, except that the amount of CEA used in binding to the liposomes was 1% relative to the lipids. The amount of CEA bonded to the liposomes was 0.2% relative to the lipids. Furthermore, the reactivity to cancer cells was investigated in the same manner as in Example 2 by using human stomach cancer cell MKN45 and free CEA. As a result, as shown in Fig. 2, almost no decrease in the reactivity to the cancer cells was observed even in the presence of free CEA.

### Example 4: Preparation of DXR-including 1-3-1 antibody-bonded liposome

In an amount of 74.8 mg of S-acetylthioglycolic acid N-hydroxysuccinimide ester (Sigma) and 41 µL of triethylamine were added to 1 g of poly(ethylene glycol)-bis-ω-amino-a-carboxyl (average molecular weight of PEG: 3,400, Shearwater Polymers, Inc) dissolved in 10 mL of methylene chloride. After dissolution with stirring, the solution was further added with 10 mg S-acetylthioglycolic acid N-hydroxysuccinimide, and allowed to react at room temperature for 3.5 hours with stirring. Progress of the reaction was monitored by a shift of a low Rf spot of the added poly(ethylene glycol)-bis- ω-amino-α -carboxyl to a high Rf (about 0.6) in TLC (chloroform/methanol = 85/10, color development with iodine, TLC was henceforth performed under the same condition).

The reaction product obtained by evaporating the solvent under nitrogen was added with 10 mL of chloroform and dissolved in the solvent. The sample was subjected to a pretreatment by addition to sep-pak (SILICA PLUS, Wates) swelled with chloroform and successive elution with chloroform/methanol (4/1 (v/v)). The solvent was evaporated again under nitrogen, and the residue was dissolved in chloroform and applied on a silica gel column (Lobaar column, LiChroprep Si60, 25 × 310 cm, Kanto Kagaku). The column was washed with chloroform, developed and eluted with chloroform/methanol (85/15 (v/v)). A main product showing Rf of about 0.6 in TLC was pooled and purified. The solvent was evaporated under nitrogen to obtain 583 mg of a product. 543 mg of the product was dissolved in about 2 mL of dehydrated methylene chloride and precipitated with addition of diethyl ether. The precipitates were collected by filtration and dried under reduced pressure by using a vacuum pump. The product was dissolved in 5 mL of dry methylene chloride, added with 17.8 mg of N-hydroxysuccinimide (Sigma), and stirred for 10 minutes. The mixture was further added with 31.9 mg of N,N'-dicyclohexylcarbodiimide and allowed to react overnight at room temperature under a nitrogen atmosphere with stirring. After the precipitates were separated by filtration, the solvent was evaporated under nitrogen, and the residue was dissolved in a small amount of dehydrated methylene chloride. Ethyl ether was added to the solution, and the precipitates were collected by filtration and dried under reduced pressure by using a vacuum pump. Then, 337 mg of the target compound was obtained by TLC as a single compound (Ac-S-PEG-Suc: the compound described in Japanese Patent Application No. 10-263262, Example 1). The production of the target substance was verified by ¹H-NMR.

Ac-S-PEG-Suc dissolved in dehydrated methanol (60 mg/ml) was added to the 1-3-1 antibodies (F(ab')₂) dissolved in 50 mM phosphate buffer, 1 mM EDTA, pH 7.0 (4.2 mg/mL). Ac-S-PEG-Suc was added in an amount of 8 times in mole of the antibodies, and allowed to react at 25°C for 1 hour. The 1-3-1 antibody introduced with the PEG derivative was purified by using a SP-Sepharose column in the same manner as in Example 2, and deprotected with hydroxylamine, and an antibody having thiol group with a PEG spacer was prepared. The antibody was desalted and the buffer was exchanged with 0.1 M phosphate buffer, 1 mM EDTA, pH 6.0 by using a PD-10 column. The amount of the introduced thiol group was measured in a similar manner, and found to be 1.3 SH/antibody.

Liposomes sized to a diameter of 0.1 µm were produced in the same manner as in Example 2 except that 0.3 M citrate buffer, pH 4.0 was used instead of the CF solution. The obtained liposomes were neutralized with 1 M sodium hydroxide and added with adriamycin (Kyowa Hakko Kogyo) in an amount of 1/10 of the weight of the lipids with warming at 60°C, which allowed the adriamycin to be encapsulated almost quantitatively. These adriamycin-including liposomes were added with the aforementioned thiolated antibody in an amount of 8% by weight of the lipids, and allowed to react at 25°C for 1 hour. The liposomes were further reacted with thiolated polyethylene glycol (Japanese Patent Unexamined Publication (Kokai) No. 4-346918) to prepare immunoliposomes to which the antibodies and the polyethylene glycol bound. Further, as a control, PEG-bonded liposomes not bonded with the antibody were produced in the same manner as described above except that the thiolated antibodies were bonded.

The 1-3-1 antibody has reactivity to human colon cancer cell line DLD-1. Therefore, DLD-1 strain was used for comparison of in vitro anti-tumor effect on cancer cells of the resulting antibody-bonded and non-antibody-bonded liposomes encapsulating adriamycin.

Cells obtained from a tumor, formed by subcutaneously transplanting DLD-1 to a nude mouse, were made aliquot in microtubes and added with the antibody-bonded or non-antibody-bonded liposomes. The mixture was allowed to react at 37°C for 1 hour. After the liposome solution was removed, the cells were inoculated to a 96-well plate and cultured. When the cells not added with the liposomes became substantially confluent, the number of cells was measured by MTT assay. As a result, as shown in Fig. 3, the antibody-bonded liposomes gave higher inhibitory effect on proliferation compared with the non-antibody-bonded liposomes.

### Industrial Applicability

The ligand-bonded complex of the present invention does not substantially bind to a free target such as a free antigen, but can specifically react with a target such as a cancer cell. Therefore, said complex enables, for example, efficient targeting therapy of tumors.

## Claims

1. A ligand-bonded complex in which a microparticle is directly or indirectly bonded to a ligand having affinity for a target substance, wherein the affinity of the ligand is sufficient to allow substantially specific binding of the ligand-bonded complex to a non-free target even in the presence of a free target.

2. The ligand-bonded complex according to claim 1, wherein two or more molecules of a single kind of the ligand having substantially the same affinity are bonded to one microparticle.

3. The ligand-bonded complex according to claim 2, wherein an amount of the ligand is sufficient for reaction with the non-free target.

4. The ligand-bonded complex according to any one of claims 1 to 3, wherein the ligand is directly bonded to the microparticle.

5. The ligand-bonded complex according to any one of claims 1 to 4, wherein a water-soluble macromolecule is bonded to the microparticle.

6. The ligand-bonded complex according to any one of claims 1 to 5, wherein a part of or all of the ligand molecules are indirectly bonded to the microparticle by means of a water-soluble macromolecule.

7. The ligand-bonded complex according to claim 5 or claim 6, wherein the water-soluble macromolecule is a polyalkylene glycol.

8. The ligand-bonded complex according to claim 5 or claim 6, wherein the water-soluble macromolecule is polyethylene glycol.

9. The ligand-bonded complex according to any one of claims 1 to 8, wherein the microparticle is selected from the group consisting of a low molecular drug, a marker molecule, a protein, a micelle, and a liposome.

10. The ligand-bonded complex according to claim 9, wherein the microparticle is a liposome.

11. The ligand-bonded complex according to claim 10, wherein the liposome encapsulates an active principle of a medicament.

12. The ligand-bonded complex according to claim 11, wherein the medicament is an anti-tumor agent.

13. The ligand-bonded complex according to any one of claims 1 to 12, wherein the ligand is an antibody.

14. The ligand-bonded complex according to claim 13, wherein the antibody is an anti-tumor antibody.

15. The ligand-bonded complex according to claim 14, wherein the antibody is bonded by means of a wafer-soluble macromolecule to a liposome encapsulating an anti-tumor agent.

16. The ligand-bonded complex according to any one of claims 1 to 15, wherein dissociation constant between the target and one ligand is E-8 M or more.

17. The ligand-bonded complex according to any one of claims 1 to 15, wherein dissociation constant between the target and one ligand is E-7 M or more.

18. A pharmaceutical composition comprising a ligand-bonded complex according to any one of claims 1 to 17.
